(19) **Europäisches Patentamt / European Patent Office / Office européen des brevets**

(11) **EP 3 188 715 B1**

(12) **FASCICULE DE BREVET EUROPEEN**

(45) Date de publication et mention de la délivrance du brevet:
**13.05.2020 Bulletin 2020/20**

(21) Numéro de dépôt: **15742183.5**

(22) Date de dépôt: **27.07.2015**

(51) Int Cl.:
*A61K 9/08* (2006.01)        *A61K 9/107* (2006.01)
*A61K 47/44* (2017.01)

(86) Numéro de dépôt international:
**PCT/EP2015/001541**

(87) Numéro de publication internationale:
**WO 2016/023617 (18.02.2016 Gazette 2016/07)**

(54) **EMULSION SOUS FORME HUILE-DANS-EAU**

Ö/W EMULSION

OIL-IN-WATER EMULSION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA**

(30) Priorité: **13.08.2014 FR 1401866**

(43) Date de publication de la demande:
**12.07.2017 Bulletin 2017/28**

(73) Titulaires:
• **Urgo Recherche Innovation et Développement**
  **21300 Chenove (FR)**
• **Hcp Healthcare Asia Pte.Ltd**
  **Singapore 138623 (SG)**

(72) Inventeurs:
• **GENTILHOMME, Marylin**
  **21000 Dijon (FR)**
• **ROY, Amélie**
  **21000 Dijon (FR)**

(74) Mandataire: **Hurtiger, Myriam**
  **Laboratoires URGO**
  **Service Brevets**
  **42, rue de Longvic**
  **21300 Chenôve (FR)**

(56) Documents cités:
**WO-A1-2011/060084      CN-A- 1 711 859**
**US-A1- 2008 274 175     US-A1- 2013 115 201**

**Description**

DOMAINE TECHNIQUE

La présente invention a pour objet une émulsion sous forme huile-dans-eau (H/E) comprenant de l'huile naturelle d'origine végétale comprenant des acides gras type Oméga 3, du fer et de la vitamine B3.

ARRIERE PLAN

Dickinson (1988) définit une émulsion comme un système hétérogène contenant un liquide dispersé dans un autre, sous forme de gouttelette de taille colloïdale ou microscopique. Plus généralement, on peut définir une émulsion comme la dispersion d'une phase non miscible dans une autre, quel que soit son état physique. Les émulsions sont formées par apport d'énergie, correspondant à l'énergie nécessaire pour créer une aire interfaciale plus grande. Par conséquent ces systèmes ne sont pas thermodynamiquement stables. La stabilité des émulsions est donc leur principal problème.

De plus des émulsions servant de vecteurs à des composés sont encore plus problématiques. En effet, aux problèmes de stabilité de l'émulsion elle-même, s'ajoutent les problèmes de stabilité de chacun des composés compris dans ladite émulsion.

Parmi les composés les plus fréquemment retrouvés dans des émulsions, on peut citer :

- Les vitamines
- Les acides gras
- Les minéraux

On entend par vitamines, les substances organiques, nécessaires en faible quantité au métabolisme d'un organisme vivant, qui ne peuvent être synthétisées en quantité suffisante par cet organisme. Les vitamines sont des compléments indispensables aux échanges vitaux. Elles sont au nombre de treize et se répartissent en deux catégories :

- les vitamines liposolubles qui sont absorbées en même temps que les graisses et stockées. Elles sont solubles dans les solvants organiques. Ce sont les vitamines A, D, E et K.
- les vitamines hydrosolubles qui ne sont pas stockées de manière prolongée et qui sont excrétées dans les urines quand leur apport est excédentaire. Elles sont solubles dans l'eau. Il s'agit des vitamines C, B1, B2, B3 (PP), B5, B6, B8, B9, B12.

On entend par acides gras, les molécules organiques comprenant une chaîne carbonée terminée par un groupement carboxylique. Cette chaîne carbonée peut être dépourvue de toute double liaison et, dans ce cas, les acides gras sont dits saturés (AGS). Elle peut aussi présenter une ou plusieurs double(s) liaison(s), les acides gras sont alors désignés sous les termes de monoinsaturés (AGMI) ou polyinsaturés (AGPI). Alors que les acides gras saturés, les monoinsaturés et une partie des polyinsaturés sont synthétisés dans l'organisme, les acides gras polyinsaturés tels que les acides linoléique (Oméga 6) et alpha-linolénique (Oméga 3), doivent être apportés par l'alimentation, c'est pour ça qu'on les appelle les acides gras essentiels.

On entend par minéral, toute substance normalement inorganique, plus rarement organique, formée naturellement ou synthétisée artificiellement, définie par sa composition chimique et l'agencement de ses atomes selon une périodicité et une symétrie précises qui se reflètent dans le groupe d'espace et dans le système cristallin du minéral.

Dans le cas objet de la présente invention, l'émulsion sous forme huile-dans-eau (H/E) comprend de l'huile naturelle d'origine végétale contenant des vitamines, des Oméga 3, et du fer.

Il est à noter que le fer est un élément extrêmement sensible à l'oxydation et à la complexation : les conditions de préparation et de stockage des aliments, les variations de pH, la présence d'autres micronutriments peuvent influencer considérablement sa biodisponibilité (Jackson & Lee, 1992 ; Hurrell, 1997 ; Dary, 2002).

De plus, au problème d'oxydation du fer s'ajoute celui de l'huile. En effet, les huiles s'oxydent facilement. Lorsque de l'oxygène se dépose sur une molécule de lipide, des peroxydes se forment. La lumière, en particulier rayonnement UV, l'apport d'oxygène par l'air ambiant, la température de stockage ou encore la teneur en ions métalliques provenant de la culture et du traitement sont des conditions qui favorisent la réaction d'oxydation.

Il existe donc un réel besoin pour une émulsion, contenant des vitamines à la fois liposolubles et hydrosolubles, qui soit stable au stockage lors du procédé de fabrication mais aussi après conditionnement dudit produit, et ce encore plus après une première utilisation du produit par le consommateur.

Le brevet CN 1 711 859 A décrit un lait fermenté comprenant du fer, de la vitamine B3 ainsi que des acides gras de type oméga 3 compris dans une huile naturelle d'origine végétale.

Les demandes de brevet US2008/274175 et US 2013/115201 décrivent des émulsions ou des liquides dans lesquels sont mélangés des huiles comprenant des acides gras de type oméga 3. Ces compostions peuvent éventuellement contenir de la vitamine B3 et du fer.

La demande de brevet WO 2011/060084 porte quant à elle sur des compositions comprenant des acides gras de type omega en association avec des agents stabilisants.

Cependant, aucune de ces demandes de brevets ne suggère d'introduire le fer sous forme chélatée dans la composition.

Pour résoudre le problème de stabilité des émulsions, le brevet EP 1 320 356 décrit un sirop ayant une longue durée de vie qui se présente sous la forme d'une émulsion huile dans-eau réalisée en mélangeant une phase aqueuse contenant des vitamines hydrosolubles, un agent gélifiant et un épaississant, avec une phase huile contenant les vitamines liposolubles et un tensioactif. De par la présence du tensioactif, cette émulsion peut être considérée comme stable.

Cependant, les propriétés inhérentes à la nature d'un tensioactif (caractère amphiphile, modification de tension de surface...) peuvent conférer à ce dernier un caractère toxique ou écotoxique, notamment quand ils se retrouvent au contact d'une muqueuse (par exemple, la muqueuse gastrique), rendant cette dernière alors plus facilement pénétrable par de nombreux toxiques.

Ainsi il apparait nécessaire de disposer d'une composition du type émulsion, dont la stabilité est améliorée, et ce sans faire intervenir nécessairement un tensioactif.

De façon tout à fait surprenante, cette problématique se trouve résolue par la réalisation d'une émulsion stable et continue, se trouvant de préférence sous forme huile-dans-eau (H/E), ladite émulsion comprenant de 5 à 15 %, en poids par rapport au poids total de l'émulsion, d'huile naturelle d'origine végétale comprenant des acides gras de type Oméga 3, de 0,1 à 1 %, en poids par rapport au poids total de l'émulsion, de fer sous forme chélaté et de 0,05 à 0,2 %, en poids par rapport au poids total de l'émulsion, de vitamines du groupe B.

La présente invention a également pour objet l'utilisation de ladite émulsion telle que décrite pour le développement cognitif, l'aide à la concentration, améliorer la mémoire, la dépression, la santé cardiovasculaire ou encore la vision.

## PRESENTATION GENERALE

Une alimentation saine et équilibrée est supposée permettre de satisfaire aux apports journaliers recommandés (AJR) en matière de vitamines et minéraux. Toutefois, le mode d'alimentation moderne peut parfois induire certaines carences alimentaires.

Dans ce contexte, la demanderesse a mis au point une émulsion sous forme huile-dans-eau, stable et continue, réalisée en mélangeant une phase aqueuse et une phase lipidique comprenant de l'huile naturelle d'origine végétale riche en acides gras Oméga 3, du fer sous forme chélaté et de la vitamine B3.

Ainsi l'invention permet, entre autre, de couvrir les besoins quotidiens de l'enfant en minéraux et en vitamines, pour compenser les insuffisances dues notamment à une alimentation déséquilibrée et au rythme de vie. Selon l'invention, l'émulsion où la phase aqueuse est dispersée dans la phase lipidique comprend entre 5 à 15 % d'huile naturelle d'origine végétale possédant des acides gras de type Oméga 3, entre 0,1 à 1 % de fer sous forme chélaté choisi parmi l'éthylènediaminetétracétate de fer et de sodium (NaFe-EDTA) ou le bisglycinate de fer (Fe-BIS), de préférence l'éthylènediaminetétracétate de fer et de sodium (NaFe-EDTA) et entre 0,05 à 0,2 % de vitamine B3 et au moins un émulsifiant de type huile dans eau (H/E) choisi parmi un polysorbate et un sucroester, de préférence du laurate de sorbitan éthoxylé à 20 moles d'oxyde d'éthylène et/ou du palmitate de saccharose.

Selon l'invention, l'émulsion comprend de l'huile naturelle d'origine végétale riche en acide gras Oméga-3 choisie parmi les huiles de colza, de noix, de soja, de pérille (*Perilla frutescens*), de lin, de cameline, de chanvre, de graines de citrouille ou de germe de blé.

De préférence, l'huile naturelle d'origine végétale riche en acide gras Oméga 3 est de l'huile de cameline.

Comme dit précédemment, le fer est sensible à l'oxydation et à la complexation : les conditions de préparation et de stockage des aliments, les variations de pH, la présence d'autres micronutriments peuvent influencer considérablement sa biodisponibilité.

La solution proposée par la présente invention consiste à utiliser des sels qui maintiennent le fer dans un état « protégé », tels que les chélates de fer.

Avantageusement le fer sous forme chélaté est choisi parmi le bisglycinate de fer (Fe-BIS) ou l'éthylènediaminetétracétate de fer et de sodium (NaFe-EDTA). De préférence, l'éthylènediaminetétracétate de fer et de sodium (NaFe-EDTA) est utilisé dans la présente invention.

Selon un mode de mise en œuvre de l'invention, l'émulsion comprend de 5 à 15 %, en poids par rapport au poids total de l'émulsion, d'huile de cameline, de 0,1 à 1 %, en poids par rapport au poids total de l'émulsion, d'éthylènediaminetétracétate de fer et de sodium (NaFe-EDTA) et de 0,05 à 0,2 %, en poids par rapport au poids total de l'émulsion, de vitamine B3.

De manière préférentielle, l'émulsion comprend de 8 à 12 %, en poids par rapport au poids total de l'émulsion, d'huile de cameline, 0,13 à 0,17 %, en poids par rapport au poids total de l'émulsion, d'éthylènediaminetétracétate de fer et de sodium (NaFe-EDTA) et 0,08 à 0,1 %, en poids par rapport au poids total de l'émulsion, de vitamine B3.

Selon l'invention, l'émulsion comprend des agents émulsifiants de type huile dans eau (H/E) afin de la stabiliser et choisi parmi un polysorbate et un sucroester.

Par agent émulsifiant du type huile dans eau, on désigne des agents émulsifiants possédant une valeur HLB suffisamment élevée pour fournir des émulsions huile dans l'eau tels que, par exemple, les esters de sorbitan éthoxylés comme l'oléate de sorbitan éthoxylé à 20 moles d'oxyde d'éthylène, le laurate de sorbitan éthoxylé à 20 moles d'oxyde d'éthylène commercialisés par la société SEPPIC respectivement sous les noms MONTANOX™ 80 B, et MONTANOX™ 20, l'alcool laurique éthoxylé à 7 moles d'oxyde d'éthylène, l'huile de ricin éthoxylée à 40 moles d'oxyde d'éthylène, commercialisés par la société SEPPIC sous le nom SIMULSOL™ P7 et SIMULSOL™ OL 50, les nonylphénols éthoxylés et/ou un sucroester.

De préférence, les agents émulsifiants seront choisis parmi un oléate de sorbitan éthoxylé à 20 moles d'oxyde d'éthylène (MONTANOX 80 B), un laurate de sorbitan éthoxylé à 20 moles d'oxyde d'éthylène (MONTANOX 20) et/ou un ester gras de saccharose et plus préférentiellement du laurate de sorbitan éthoxylé à 20 moles d'oxyde d'éthylène (MONTANOX 20) et/ou un sucroester.

De manière préférentielle, ladite émulsion comprend de 0,05 à 0,15 %, en poids par rapport au poids total de l'émulsion de polysorbate, de préférence de 0,08 à 0,10 %, en poids par rapport au poids total de l'émulsion de polysorbate et de 1 à 10 %, en poids par rapport au poids total de l'émulsion de sucrosester, de préférence de 2 à 5 %, en poids par rapport au poids total de l'émulsion de sucroester.

Afin de caractériser une huile soumise à l'oxydation et ainsi caractériser sa stabilité il existe l'indice de peroxyde (IP).

Les peroxydes sont des produits primaires de l'oxydation de matières grasses. La mesure de l'indice de peroxyde est un titrage. La matière grasse à analyser est mise en solution dans un mélange de chloroforme et d'acide acétique. De l'iodure de potassium (KI) est ajouté. Ce KI réagit avec les peroxydes et de l'iode ($I_2$) se forme. Cet iode est ensuite dosé par réaction avec $Na_2S_2O_3$.

Pour caractériser la stabilité de l'huile, les inventeurs ont déterminé l'indice de peroxyde et ils ont dosé l'acide alpha-linolénique au cours du temps pour des conditions de température et d'humidité différentes.

Selon la présente invention, l'huile végétale a un indice de peroxyde (IP) inférieur ou égal à 3 $mEqO_2$/kg pendant une durée d'au moins 3 mois à une température de 40°C.

De préférence l'indice de peroxyde (IP) inférieur ou égal à 2 $mEqO_2$/kg et encore plus préférentiellement inférieur ou égal à 1,5 $mEqO_2$/kg pendant une durée d'au moins 3 mois à une température de 40°C.

Selon l'invention, la vitamine B3 est choisie parmi la niacine (acide nicotinique ou l'acide pyridine-3-carboxylique) et nicotinamide (niacinamide) et ses sels et esters.

Avantageusement, ladite émulsion comprend des vitamines du groupe D, de préférence de la vitamine D3, et/ou des vitamines du groupe E, de préférence de l'acétate d'alpha-tocophérol.

La vitamine D est une molécule signal clé dans la régulation du métabolisme de calcium dans l'organisme et dans la minéralisation du tissu osseux dont elle influence la densité et le remodelage. Le taux physiologique de 25-hydroxyvitamine D est indispensable chez les enfants pour la croissance des os.

La vitamine E est un antioxydant, capable de retarder ou ralentir le rancissement, ou l'apparition de flaveurs indésirables dues à l'oxydation.

Ainsi, la vitamine E permet augmenter la durée de vie d'un aliment, réduire les pertes en vitamines ou en résidus d'acides gras essentiels. Néanmoins de fortes concentrations pourraient être responsables d'un effet pro-oxydant, comme cela a été démontré pour l'alpha-tocophérol (Cillard & Cillard, 1980 ; Huang *et al.,* 1994).

De préférence, l'émulsion comprend de 0,01 à 0,05 %, en poids par rapport au poids total de l'émulsion, de vitamine D3 et plus préférentiellement de 0,02 à 0,04 %, en poids par rapport au poids total de l'émulsion. De préférence l'émulsion comprend de 0,2 à 0,6 %, en poids par rapport au poids total de l'émulsion, de vitamine E et plus préférentiellement de 0,3 à 0,5 %, en poids par rapport au poids total de l'émulsion.

L'invention a également pour objet l'utilisation d'une émulsion comprenant de l'huile de cameline et du fer pour le développement cognitif, l'aide à la concentration, améliorer la mémoire, la dépression, la santé cardiovasculaire et/ou la vision.

DESCRIPTION DETAILLEE D'EXEMPLE(S)

L'invention est illustrée par l'exemple suivant. Il s'agit d'exemples de émulsion telle que décrite dans la présente invention, dans un flacon de 150 ml:
EXEMPLE 1 :

TABLEAU 1

| DENOMINATION | dosage en mg/flacon | % | actif |
|---|---|---|---|
| HUILE DE CAMELINE | 17850,1 | 12 | ALA |
| MONTANOX 20 | 14,7 | 0,009 | |
| ESTER GRAS DE SACCHAROSE | 49500 | 3 | |
| VITAMINE B3 PP | 157,7 | 0, 1 | Vitamine B3 |
| FER SODIUM EDTA | 242,3 | 0,16 | Fer |
| VITAMINE D3 | 39,0 | 0,02 | Vitamine D |
| VITAMINE E | 349,3 | 0,2 | $\alpha$-tocophérol |

EXEMPLE 2 :

TABLEAU 2

| DENOMINATION | dosage en mg/flacon | % | actif |
|---|---|---|---|
| HUILE DE CAMELINE | 18000,0 | 11 | ALA |
| MONTANOX 20 | 14,7 | 0,009 | |
| ESTER GRAS DE SACCHAROSE | 4950,0 | 3 | |
| VITAMINE B3 PP | 157,7 | 0,1 | Vitamine B3 |
| FER SODIUM EDTA | 242,3 | 0,16 | Fer |
| VITAMINE D3 | 39,0 | 0,02 | Vitamine D |
| VITAMINE E | 349,3 | 0,2 | $\alpha$-tocophérol |

EXEMPLE 3 :

TABLEAU 3

| DENOMINATION | dosage en mg/flacon | % | actif |
|---|---|---|---|
| HUILE DE CAMELINE | 13090,9 | 8 | ALA |
| MONTANOX 20 | 14,7 | 0,009 | |
| ESTER GRAS DE SACCHAROSE | 4950,0 | 3 | |
| VITAMINE B3 PP | 157,7 | 0,1 | Vitamine B3 |
| FER SODIUM EDTA | 242,3 | 0,16 | Fer |
| VITAMINE D3 | 39,0 | 0,02 | Vitamine D |
| VITAMINE E | 349,3 | 0,2 | $\alpha$-tocophérol |

Le niveau d'oxydation de l'huile peut s'évaluer à partir de la mesure de l'indice de peroxyde qui détermine la concentration en hydroperoxydes d'acides gras, dits composés primaires d'oxydation, pouvant se former dans l'huile en présence d'oxygène. De plus, les inventeurs ont dosé l'acide alpha-linolénique au cours du temps pour des conditions de température et d'humidité différentes.

La méthode dite « au chloroforme » selon règlement CEE 2568/91-Annexe III pour déterminer l'indice de peroxyde, a été utilisée dans la présente invention.

L'indice de peroxyde représente la quantité des substances de l'échantillon (exprimée en milliéquivalents d'oxygène actif par kilogramme) qui oxydent l'iodure de potassium dans les conditions de travail décrites.

La prise d'essai en solution dans un mélange acide acétique et chloroforme est traitée par une solution d'iodure de potassium. L'iode libéré est titré avec une solution de thiosulfate de sodium.

L'échantillon est prélevé et stocké hors de la lumière, conservé au frais et enfermé dans des conteneurs de verre remplis entièrement et fermés hermétiquement à l'aide de bouchons de liège ou de verre rodé.

MODE OPÉRATOIRE

L'essai doit être réalisé sous une lumière diffuse (lumière du jour) ou artificielle. Dans une cuillère en verre (5.1) ou, à défaut, dans une fiole (5.2), peser, à 0,001 gramme près, une des masses de l'échantillon visées dans le tableau ci-après en fonction de l'indice de péroxyde prévu.

TABLEAU 4

| Indice de peroxyde prévu (en meq $0_2$/kg) | Poids de la prise d'essai (en g) |
|---|---|
| de 0 à 12 | de 5,0 à 2,0 |
| de 12 à 20 | de 2,0 à 1,2 |
| de 20 à 30 | de 1,2 à 0,8 |
| de 30 à 50 | de 0,8 à 0,5 |
| de 50 à 90 | de 0,5 à 0,3 |

Déboucher une fiole et introduire la cuiller en verre contenant la prise d'essai. Ajouter 10 millilitres de chloroforme. Dissoudre rapidement la prise d'essai en agitant. Ajouter 15 millilitres d'acide acétique puis 1 millilitre de solution d'iodure de potassium. Remettre le bouchon rapidement, agiter pendant une minute et laisser reposer pendant exactement 5 minutes à l'abri de la lumière et à une température de 15 à 25 °C.

Ajouter environ 75 millilitres d'eau distillée. Titrer l'iode libéré avec la solution de thiosulfate de sodium (solution 0,002 N, si les indices prévus sont inférieurs à 12, et 0,001 N, s'ils sont supérieurs à 12) en agitant vigoureusement et en employant la solution d'amidon comme indicateur.

Effectuer deux déterminations sur le même échantillon.

Effectuer simultanément un essai à blanc. Si le résultat de ce dernier excède 0,05 millilitre de solution de thiosulfate de sodium 0,001 N, remplacer les réactifs impurs.

RÉSULTATS

L'indice de peroxyde (IP), exprimé en milliéquivalents d'oxygène actif par kilogramme, est fourni par la formule:

$$IP = \frac{V \times T \times 1000}{m}$$

V: = nombre de millilitres de solution de thiosulfate de sodium normalisée (6.4) utilisé pour l'essai, corrigé en fonction des résultats de l'essai à blanc;
T: = facteur de normalité exact de la solution de thiosulfate de sodium (6.4) utilisée;
m: = poids (en grammes) de la prise d'essai.

On a obtenu les résultats suivants :

TABLEAU 5

| CONDITIONS TEMPERATURE / HUMIDITE | TEMPS EN MOIS | INDICE DE PEROXYDE |
|---|---|---|
| **EXEMPLE 1** | | |
| Ambiante | 0 | 2,7 |
| 40 °C / 75% | 1,5 | 2,6 |
| 40 °C / 75% | 3 | 1,6 |
| **EXEMPLE 2** | | |
| Ambiante | 0 | 2,5 |

(suite)

| EXEMPLE 2 | | |
|---|---|---|
| 40 °C / 75% | 1,5 | 2,4 |
| 40 °C / 75% | 3 | 1,5 |
| EXEMPLE 3 | | |
| Ambiante | 0 | 1,8 |
| 40 °C / 75% | 1,5 | 1,7 |
| 40 °C / 75% | 3 | 1,0 |

Les résultats obtenus par la méthode au chloroforme montrent une valeur de l'indice de peroxyde inférieure à 2,6 $mEqO_2$/kg pour les trois exemples après 1 mois et demi de conservation à 40°C et une valeur de l'indice de peroxyde inférieure à 1,5 $mEqO_2$/kg pour l'échantillon après 3 mois de conservation à 40°C.

De plus la quantité d'acide alpha-linolénique a été dosée au cours du temps pour des conditions de température et d'humidité différentes afin de vérifier que la concentration en acide alpha linolénique reste conforme aux spécifications et ainsi démontrer la stabilité de l'émulsion.

TABLEAU 6

| CONDITIONS TEMPERATURE / HUMIDITE | TEMPS EN MOIS | ACIDE ALPHA-LINOLENIQUE (mg/10 ml) |
|---|---|---|
| EXEMPLE 1 | | |
| Ambiante | 0 | 463,44 |
| 25 °C / 60% | 3 | 441,56 |
| 40 °C / 75% | 3 | 359,93 |
| EXEMPLE 2 | | |
| Ambiante | 0 | 424,82 |
| 25 °C / 60% | 3 | 404,77 |
| 40 °C / 75% | 3 | 329,94 |
| EXEMPLE 3 | | |
| Ambiante | 0 | 308,96 |
| 25 °C / 60% | 3 | 294,37 |
| 40 °C / 75% | 3 | 239,96 |

Selon la présente invention, l'émulsion est stable comme quand la concentration en acide alpha-linolénique est supérieure à 200 mg / 10 ml.

Ainsi, on constate que la concentration en acide alpha-linolénique, pour les trois exemples, au cours du temps pour des conditions de température et d'humidité différentes reste supérieure à 200 mg / 10 ml.

Par conséquent les résultats obtenus démontrent, par l'intermédiaire de l'indice de peroxyde et la concentration en acide alpha-linolénique, qu'une composition sous forme d'émulsion huile-dans-eau réalisée selon l'invention est stable dans le temps.

**Revendications**

1. Emulsion, sous forme huile-dans-eau, comprenant :

   - De 5 à 15 %, en poids par rapport au poids total de l'émulsion, d'huile naturelle d'origine végétale comprenant des acides gras de type Oméga 3 ;
   - De 0,1 à 1 %, en poids par rapport au poids total de l'émulsion, de fer sous forme chélaté choisi parmi l'éthylènediaminetétracétate de fer et de sodium (NaFe-EDTA) ou le bisglycinate de fer (Fe-BIS), de préférence l'éthylènediaminetétracétate de fer et de sodium (NaFe-EDTA) ; et
- De 0,05 à 0,2 %, en poids par rapport au poids total de l'émulsion, de vitamine B3
- au moins un émulsifiant de type huile dans eau (H/E) choisi parmi un polysorbate et un sucroester, de préférence du laurate de sorbitan éthoxylé à 20 moles d'oxyde d'éthylène et/ou du palmitate de saccharose.

**2.** Emulsion selon l'une quelconque des revendications précédentes, **caractérisée en ce que** l'huile naturelle d'origine végétale comprenant des acides gras Oméga type 3 est choisie parmi les huiles de colza, de noix, de soja, de pérille (*Perilla frutescens*), de lin, de cameline, de chanvre, de graines de citrouille ou de germe de blé.

**3.** Emulsion selon l'une quelconque des revendications précédentes, **caractérisée en ce que** ladite émulsion comprend :

- De 8 à 12 %, en poids par rapport au poids total de l'émulsion, d'huile de cameline ;
- De 0,13 à 0,17 %, en poids par rapport au poids total de l'émulsion, d'éthylènediaminetétracétate de fer et de sodium (NaFe-EDTA); et
- De 0,08 à 0,1 %, en poids par rapport au poids total de l'émulsion, de vitamine B3.

**4.** Emulsion selon la revendication 5 ou 6, **caractérisée en ce que** ladite émulsion comprend de 0,05 à 0,15 %, en poids par rapport au poids total de l'émulsion, de polysorbate et 1 à 10 %, en poids par rapport au poids total de l'émulsion, de sucroester, de préférence de 0,08 à 0,1 %, en poids par rapport au poids total de l'émulsion, de polysorbate et 2 à 5 %, en poids par rapport au poids total de l'émulsion, de sucroester.

**5.** Emulsion selon l'une quelconque des revendications précédentes, **caractérisée en ce que** l'huile naturelle d'origine végétale comprenant des acides gras Oméga type 3 a un indice de peroxyde (IP) inférieur ou égal à 3 mEqO$_2$/kg pendant une durée d'au moins 3 mois à une température de 40°C.

**6.** Emulsion selon l'une quelconque des revendications précédentes, **caractérisée en ce que** ladite émulsion comprend des vitamines du groupe D, de préférence de la vitamine D3, et/ou des vitamines du groupe E, de préférence de l'acétate de tocophérol.

**7.** Emulsion selon l'une quelconque des revendications précédentes comprenant de l'huile de cameline et du fer pour son utilisation pour le développement cognitif, l'aide à la concentration, améliorer la mémoire, la dépression, la santé cardiovasculaire et/ou la vision.

**Patentansprüche**

**1.** Öl-in-Wasser-Emulsion mit folgendem Inhalt:

- 5 bis 15 % Massenanteil (an der gesamten Emulsion) pflanzliche Öle natürlicher Herkunft, die Omega-3-Fettsäuren enthalten
- 0,1 bis 1 % Massenanteil (an der gesamten Emulsion) in Chelatkomplexen gebundenes Eisen, entweder Eisen-Natrium-Ethylendiamintetraacetat (NaFe-EDTA) oder Eisen(II)-glycinat (FeBI5), vorzugsweise Eisen-Natrium-Ethylendiaminteraacetat (NaFe-EDTA)
und
- 0,05 bis 0,2 % Massenanteil (an der gesamten Emulsion) Vitamin B3
- mindestens ein Öl-in-Wasser-Emulgator (O/W), entweder ein Polysorbat oder ein Zuckerester, vorzugsweise ein Sorbitanmonolaurat, das mit 20 Mol Ethylenoxid ethoxyliert wurde, und/oder Saccharosepalmitat

**2.** Emulsion gemäß einem der vorstehenden Ansprüche, jedoch dadurch genauer gekennzeichnet, dass das Öl pflanzlicher Herkunft mit Omega-3-Fettsäuregehalt aus folgenden Ölen gewählt wird: Raps, Walnuss, Soja, Perilla (*Perilla frutescens*), Lein, Leindotter, Hanf, Kürbis oder Weizenkeim

**3.** Emulsion gemäß einem der vorstehenden Ansprüche, jedoch dadurch genauer gekennzeichnet, dass die Emulsion Folgendes beinhaltet:

- 8 bis 12 % Massenanteil (an der gesamten Emulsion) Leindotteröl

- 0,13 bis 0,17 % Massenanteil (an der gesamten Emulsion) Eisen-Natrium-Ethylendiaminetatraacetat (NaFe-EDTA)
- 0,08 bis 0,1 Massenanteil (an der gesamten Emulsion) Vitamin B3

**4.** Emulsion gemäß Anspruch 5 oder 6, dadurch genauer gekennzeichnet, dass die Emulsion 0,05 bis 0,15 % Massenanteil (an der gesamten Emulsion) Polysorbate und 1 bis 10 % Massenanteil (an der gesamten Emulsion) Zuckerester enthält, vorzugsweise 0,08 bis 0,1 % Massenanteil (an der gesamten Emulsion) Polysorbate und 2 bis 5 % Massenanteil (an der gesamten Emulsion) Zuckerester

**5.** Emulsion gemäß einem der voranstehenden Ansprüche, dadurch genauer gekennzeichnet, dass das natürliche Öl pflanzlicher Herkunft für eine Dauer von mindestens 3 Monaten bei einer Temperatur von 40 °C Omega-3-Fettsäuren mit einer Peroxidzahl (POZ) kleiner oder gleich 3 mEqO$_2$/kg enthält

**6.** Emulsion gemäß einem der voranstehenden Ansprüche, dadurch genauer gekennzeichnet, dass die Emulsion Vitamine der D-Gruppe - vorzugsweise Vitamin D3 - und Vitamine der E-Gruppe - vorzugsweise Tocopherylacetat - enthält

**7.** Emulsion gemäß einem der voranstehenden Ansprüche, die Leindotteröl und Eisen enthält, damit sie für die kognitive Entwicklung, als Konzentrationshilfe, zur Verbesserung des Gedächtnisses, gegen Depressionen, für die Gesundheit des Herz-Kreislaufsystems und/oder zur Erhaltung der Sehkraft eingesetzt werden kann

**Claims**

**1.** An oil-in-water emulsion, comprising:

- Between 5 and 15%, by weight of the emulsion's total weight, of natural vegetable oil containing Omega-3 type fatty acids;
- Between 0.1 and 1%, by weight of the emulsion's total weight, of iron in a chelate form chosen from the following: iron and sodium ethylene diamine tetraacetates (NaFe-EDTA) or ferrous bisglycinate (Fe-BI5), with a preference for iron and sodium ethylene diamine tetraacetates (NaFe-EDTA);
and
- Between 0.05 and 0.2%, by weight of the emulsion's total weight, of vitamin B3
- at least an oil-in-water (o/w) emulsifier selected from polysorbates and sucroesters, preferably sorbitan laurate ethoxylated with 20 moles of ethylene oxide and/or saccharose palmitate.

**2.** Emulsion according to any one of the aforementioned claims, **characterised by** a natural vegetable oil containing Omega-3 type fatty acids selected from any of the following sources: rapeseed, walnut, soya, perilla (*Perilla frutescens*), flax, camelina, hemp, pumpkin seeds and wheat germ.

**3.** Emulsion according to any one of the aforementioned claims, **characterised by** the presence in the said emulsion of:

- Between 8 and 12%, by weight of the emulsion's total weight, of camelina oil;
- Between 0.13 and 0.17%, by weight of the emulsion's total weight, of iron and sodium ethylene diamine tetraacetates (NaFe-EDTA); and
- Between 0.08 and 0.1%, by weight of the emulsion's total weight, of vitamin B3.

**4.** Emulsion according to claim 5 or 6, **characterised by** the said emulsion containing between 0.05 and 0.15%, by weight of the emulsion's total weight, of polysorbate and between 1 and 10%, by weight of the emulsion's total weight, of sucroester, preferably between 0.08 and 0.1%, by weight of the emulsion's total weight, of polysorbate and between 2 and 5%, by weight of the emulsion's total weight, of sucroester.

**5.** Emulsion according to any of the aforementioned claims, **characterised by** a natural vegetable oil containing Omega-3 type fatty acids with a peroxide value less than or equal to 3 mEq of O$_2$/kg over a period of at least 3 months at a temperature of 40°C.

**6.** Emulsion according to any of the aforementioned claims, **characterised by** the presence in the said emulsion of group D vitamins, preferably vitamin D3, and/or group E vitamins with a preference for tocopherol acetate.

7. Emulsion according to any of the aforementioned claims, containing camelina oil and iron for help in the following areas: cognitive development, concentration, memory improvement, depression, cardiovascular problems and/or eyesight.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- CN 1711859 A
- US 2008274175 A
- US 2013115201 A
- WO 2011060084 A
- EP 1320356 A